# EUROPEAN PATENT SPECIFICATION

(11) **EP 2 020 242 B1**
(45) Date of publication and mention of the grant of the patent: **19.10.2011**
(21) Application number: 08163006.3
(22) Date of filing: 10.08.2000
(51) Int. Cl.: A61K 41/00, A61K 47/10, A61K 47/12, A61K 47/20, A61K 47/48, C07D 311/82, A61P 17/00, A61P 17/06

(54) **Improved Topical Medicaments and Methods for Photodynamic Treatment of Disease**
Verbesserte topische Medikamente und Verfahren zur photodynamischen Krankheitsbehandlung
Médicaments topiques améliorés et procédés de traitement photodynamique de maladies

(30) Priority: 13.08.1999 US 149015 P; 09.08.2000 US 635276
(43) Date of publication of application: 04.02.2009
(62) Divisional of application: 00959216.3
(73) Proprietor: Provectus Pharmatech, Inc., Knoxville, TN 37931 (US)
(72) Inventor: Dees, Craig H, Knoxvillle, TN 37923 (US); Scott, Timothy, Knoxville, TN 37923 (US); Smolik, John, Loudon, TN 37774 (US); Wachter, Eric, Oak Ridge, TN 37830 (US); Fisher, Walter, Knoxville, TN 37922 (US)
(74) Representative: Hill, Christopher Michael

(56) References cited:
- EP-A- 0 175 617
- WO-A-00/07515
- WO-A-00/25665
- WO-A-00/37927
- WO-A-90/13296
- WO-A-93/21992
- WO-A-95/02324
- US-A- 4 973 848
- US-A- 5 556 992
- US-A- 5 576 013
- US-A- 5 773 460
- US-A- 5 780 052
- VALENZENO D P ET AL: "CELL MEMBRANE PHOTOMODIFICATION: RELATIVE EFFECTIVENESS OF HALOGENATED FLUORESCEINS FOR PHOTOHEMOLYSIS" PHOTOCHEMISTRY AND PHOTOBIOLOGY, OXFORD, GB, vol. 35, no. 3, 1 January 1982 (1982-01-01), pages 343-350, XP009066724 ISSN: 0031-8655
- DATABASE EMBASE [Online] ELSEVIER SCIENCE PUBLISHERS, AMSTERDAM, NL; KIPNIS, V.M.: "Treatment of psoriasis with ultraviolet irradiation in combination with 1% alcohol solution of eosin." XP002497501 retrieved from STN Database accession no. 1978309049 & VESTNIK DERMATOLOGII I VENEROLOGII, VOL. 54, NO. 3, PP. 70-72. ISSN: 0042-4609 CODEN: VDVEAV, 1978,
- JOSHI P C ET AL: "THE ROLE OF ACTIVE OXYGEN (1O2 AND O2-) INDUCED BY CRUDE COAL TAR AND ITS INGREDIENTS USED IN PHOTOCHEMOTHERAPY OF SKIN DISEASES" JOURNAL OF INVESTIGATIVE DERMATOLOGY, NATURE PUBLISHING GROUP, GB, vol. 82, no. 1, 1 January 1984 (1984-01-01), pages 67-73, XP008062929 ISSN: 0022-202X

## Description

### BACKGROUND OF THE INVENTION

The present invention is related to certain photodynamic, topically-applicable medicaments and their uses in the treatment of human or animal tissue using photodynamic therapy (PDT).

PDT was originally developed to treat cancer and other diseases with the promise of limiting the invasiveness of the therapeutic intervention and lessening potential collateral damage to normal, non-diseased tissue. In its simplest form, PDT is the combination of a photosensitive agent with special forms of illumination to produce a therapeutic response in certain tissues, such as a tumor. The agent attains an excited, active state when it absorbs one or more photons and then is or becomes efficacious. Key elements of a successful PDT regimen include either selective application or selective uptake of a photosensitive agent into the diseased tissue and site-specific application of the activating light. PDT agents are typically applied systemically (for example, via intravenous injection or oral administration) or via localized topical application directly to diseased tissues (for example, via topical creams, ointments, or sprays). Subsequent to administration of the agent (typically 30 minutes to 72 hours later), an activating light is applied to the disease site, locally activating the agent, and destroying the diseased tissue. Light is typically applied by direct illumination of the site, or by delivery of light energy to internal locations using a fiberoptic catheter or similar device.

Most current PDT regimens are based on systemic application of porphyrin-based agents or topical or systemic application of psoralen-based agents. Examples of porphyrin-based agents include porfimer sodium (PHOTOFRIN®), hematoporphyrin-derivative (HPD), benzoporphyrin derivative (BPD), Lutex, BOPP, 5-aminolevulinic acid (ALA), and SnET₂. PHOTOFRIN® is one of the few agents currently licensed by the U.S. FDA. Porphyrin-based agents generally are derived from complex mixtures of natural or synthetically prepared materials, and may contain components that are lipophilic. As a possible result of this lipophilicity, porphyrin-based agents have shown a slight tendency to preferentially accumulate in some tumors and other diseased tissues. However, the targeting of such agents to diseased tissue is still unacceptably low when compared to uptake in normal tissue (i.e., at most 2- 10x greater uptake in diseased tissue relative to normal tissue). The psoralens, such as 8-MOP, 5-MOP, trioxsalen, and AMT, are nucleic acid intercalators that function by impairing cellular physiology. This intercalation appears to be relatively indiscriminate in terms of tissue type, and as a result these agents also exhibit minimal specificity for diseased tissue. Thus, current agents have failed to exhibit sufficient specificity, and may exhibit additional disadvantages, including persistent systemic or localized photosensitivity, systemic or localized toxicity, and unacceptable treatment cost (due to high agent cost or excessive dosage requirements).

The inherent disadvantages of various current PDT agents and medicaments containing such agents have made acceptable PDT-based treatment of various human and animal conditions difficult or impossible. These disadvantages are particularly serious in the case of indications affecting external or internal surface or near surface tissues, where it would be desirable to have medicaments suitable for localized, selective treatment of the desired tissues. Such indications include a variety of conditions affecting the skin and related organs, the mouth and digestive tract and related organs, the urinary and reproductive tracts and related organs, the respiratory tract and related organs, as well as various other tissue surfaces, such as tissue surfaces exposed during surgery.

US 5,556,992 discloses novel rhodamine derivatives for photodynamic therapy of cancer and *in vitro* purging of leukemias.

US 5,773,460 discloses rhodamine derivatives for photodynamic therapy of cancer and *in vitro* purging of the leukemias.

US 5,780,052 discloses compositions and methods useful for inhibiting cell death and for delivering an agent into a cell.

US 5,576,013 is directed to the treatment of vascular and neoplastic tissues.

US 4,973,848 discloses a laser apparatus for concurrent analysis and treatment.

EP0175617 discloses antibody therapeutic agent conjugates.

Valenzeno et al (Photochemistry and Photobiology, Oxford, Vol. 35, No. 3, 1st January 1982, pages 343 to 350; XP009066724) discloses the relative effectiveness of halogenated fluoresceins for photohemolysis.

WO90/13296 discloses antiviral therapy using thiazine and xanthene dyes.

WO93/21992 discloses a method of disinfecting or sterilising tissues of the oral cavity or a wound or lesion in the oral cavity, which comprises applying a photosensitizing compound to said tissues, wound or lesion, and irradiating said tissues, wound or lesion with laser light.

XP002497501 discloses of treatment of psoriasis with ultraviolet irradiation.

Joshi et al (Journal of Investigative Dermatology, Vol. 82, No. 1, 1st January 1984, pages 67 to 73; XP008062929) discloses the role of active oxygen species induced by crude coal tar and its ingredients used in photochemotherapy of skin diseases.

WO00/07515 discloses an improved method for targeted topical treatment of disease.

WO00/25665 discloses a method for improved imaging and photodynamic therapy.

WO00/37927 discloses high energy phototherapeutic agents.

Therefore, it is an object of the present invention to provide new medicaments, medical uses for such medicaments based on targeted application of such medicaments thereby resulting in increased efficacy and safety and reduced cost of treatment.

### SUMMARY OF THE INVENTION

The present invention provides the use of at least one halogenated xanthene for the manufacture of a medicament for topical application, for the photodynamic treatment of diseases selected from psoriasis, pustular psoriasis, atopic dermatitis, eczematous disease and eczematous reaction;
said halogenated xanthene being dissolved in a delivery vehicle, said delivery vehicle including one or more adjuvants selected from builders, stabilizers, emulsifiers, dispersants, preservatives, buffers, electrolytes, tissue penetrating agents and tissue softening agents;
wherein said halogenated xanthene is selected from the group consisting of 4,5,6,7-Tetrabromoerythrosin and Mono-, Di-, or Tribromoerythrosin;
provided said halogenated xanthene is present in a concentration of greater than approximately 0.001% to less than approximately 20%; and
wherein said photodynamic treatment comprises activation by employing a light having a wavelength of between approximately 500 nm and 600 nm.

The present invention is thus directed to new photodynamic, topically-applicable medicaments and certain medical usesofsuch photodynamic medicaments for treatment of human or animal tissue, wherein a primary active component of such medicaments is a halogenated xanthene.

The halogenated xanthenes constitute a family of potent photosensitizers that become photoactivated upon illumination with visible wavelengths of light. Such medicaments can also be called pharmaceutical compositions or agents.

The inventors of the present invention have found that such medicaments are useful for treatment of a variety of conditions affecting the skin.

These medicaments are applied in various formulations including liquid, semisolid or aerosol delivery vehicles. Photoactivation of photoactive ingredients in such medicaments produces a desirable medical response, such as destruction of microbial infection, reduction or elimination of tissue irritation, reduction or elimination of hyperproliferative tissue, reduction or elimination of cancerous or precancerous tissue, reduction or elimination of surface or subsurface lipocytes or lipid deposits, and many other similar indications.

In a preferred embodiment, such medicaments are used for treatment of a variety of conditions affecting the skin

In another preferred embodiment, such medicaments are used for treatment of a variety of conditions related to microbial or parasitic infection.

In another preferred embodiment, such medicaments are produced in various formulations including liquid, semisolid or aerosol delivery vehicles.

### BRIEF DESCRIPTION OF THE DRAWINGS

In describing the preferred embodiments, reference is made to the accompanying drawings wherein:
FIGURE 1 (a) shows the generalized chemical structure of the halogenated xanthenes.
FIGURE 1(b) shows the chemical structure of Rose Bengal.
FIGURE 2 shows example absorbance spectra of several halogenated xanthenes.

### DETAILED DESCRIPTION OF THE PRESENTLY PREFERRED EMBODIMENTS

The present invention is directed to new photodynamic, topically-applicable medicaments and certain medical uses of such photodynamic medicaments for treatment of human or animal tissue, wherein a primary active component of such medicaments is a halogenated xanthene. Such halogenated xanthenes discussed *infra* are capable of exhibiting a desirable photodynamic effect when applied to or otherwise delivered to certain human or animal tissues, and undergo photodynamic activation in such tissues upon illumination with visible, and in particular green, light. These desirable effects include reduction or elimination of disease or other undesirable conditions, including eradication of cancerous or pre-cancerous tumors and infectious agents, and are applicable to a variety of conditions affecting the skin.

In a preferred embodiment, such medicaments are produced in various formulations including liquid, semisolid or aerosol delivery vehicles.

### 1. Properties of the preferred photoactive components and medicament formulations.

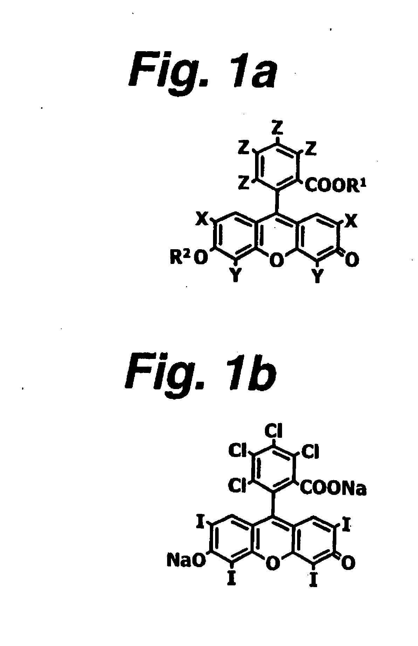

The applicants have discovered that a certain class of photoactive agents are broadly applicable for producing topically-applicable medicaments for treatment of certain human and animal tissues. These photoactive agents are referred to as halogenated xanthenes and are illustrated in Figure 1a, where the symbols X, Y, and Z represent various elements present at the designated positions, and the symbols R¹ and R² represent various functionalities present at the designated positions.

Selected chemical and physical properties (such as the chemical constituents at positions X, Y, and Z and the functionalities R¹ and R², along with molecular weight and photochemical characteristics) of representative halogenated xanthenes are summarized in attached Table 1. The general properties of this class of agents are discussed in further detail in USSN 09/130,041, filed on August 6, 1998, USSN 09/184,388, filed on November 2, 1998, and USSN 09/216,787, filed on December 21, 1998.

In general, the halogenated xanthenes are characterized by a low dark cytotoxicity (toxicity to cells or tissues in the absence of photoactivation), high light cytotoxicity (toxicity to cells or tissues upon photoactivation) and chemical and photochemical properties that are substantially unaffected by the local chemical environment or the attachment of functional derivatives at positions R¹ and R². This makes such chemical agents, and in particular medicaments formulated from such agents, excellent PDT agents for the treatment of human and animal tissues. According to the present invention a topically-applicable medicamenti sproduced that contains, as an active ingredient at a concentration of from greater than approximately 0.001% to less than approximately 20%, at least one halogenated xanthene.

The halogenated xanthenes used in the present invention are selected from one or more of:
4,5,6,7-Tetrabromoerythrosin; Mono-, Di-, or Tribromoerythrosin.

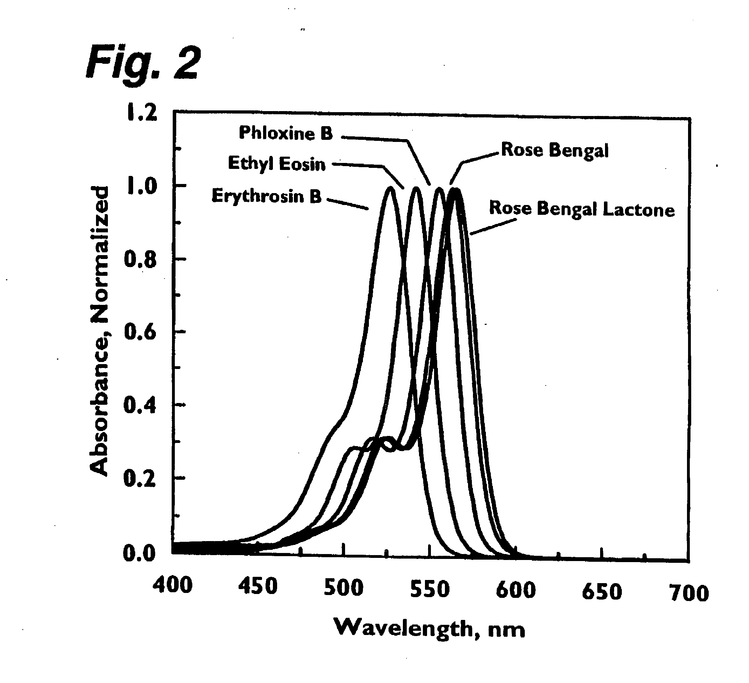

Further, as evidenced by the data shown in Table 1 and in Figure 2, it is clear that the halogenated xanthenes share common spectroscopic properties, including a high single-photon cross-section extending from approximately 500 nm to 600 nm. These properties are substantially invariant regardless of state of derivatization (for example, at positions R¹ and R²) or of chemical or biological environment. This feature facilitates photoactivation with commonly available visible light sources, such as cw or pulsed lasers or lamps, operating in the band from approximately 500 nm to 600 nm, and circumvents the need to substantively change sources if the specific photoactive component of the medicament is varied or modified, as disclosed herein. Furthermore, the inventors of the present invention have shown that the halogenated xanthenes are capable of being activated using non-linear, multi-photon excitation under certain conditions when using light in the near infrared band from approximately 700 nm to 1200 nm (using methods, such as for example, those taught in USSN 08/989,231, filed December 11, 1997 and USSN 09/096,832 filed June 12, 1998. Such excitation methods provide additional utility in activation of medicaments formulated from such agents, such as for example when it is desirable to increase the depth of photoactivation to positions substantially below an exposed tissue surface.

As an example of these desirable chemical, biochemical, and physical properties, the inventors have found that the prototypical halogenated xanthene, Rose Bengal, will accumulate preferentially in (i.e. target) some tumors and other diseased tissues and pathogens, has negligible dark cytotoxicity, high light cytotoxicity upon illumination with visible light, relatively low cost, and the ability to clear rapidly from the body.

Moreover, the inventors have discovered that the facility with which the halogenated xanthenes target specific tissues or other sites can be further optimized by attachment of specific functional derivatives at positions R¹ and R², so as to change the chemical partitioning or biological activity of the agent. For example, attachment of one targeting moiety or more at positions R¹ or R² can be used to improve targeting to specific tissues, such as cancerous tumor tissues or sites of localized infection. An example of this is esterification at position R¹ with a short aliphatic alcohol, such as n-hexanol, to produce a derivatized agent exhibiting enhanced partitioning into lipid-rich tumor tissues.

It is thus a further preferred embodiment that at least one of the at least one halogenated xanthene active ingredients includes at least one targeting moiety selected from a group that includes DNA, RNA, amino acids, proteins, antibodies, ligands, haptens, carbohydrate receptors or complexing agents, lipid receptors or complexing agents, protein receptors or complexing agents, chelators, encapsulating vehicles, short-or long-chain aliphatic or aromatic hydrocarbons, including those containing aldehydes, ketones, alcohols, esters, amides, amines, nitriles, azides, or other hydrophilic or hydrophobic moieties. A further example of this embodiment is derivatization of Rose Bengal with a lipid (at position R¹, via esterification), so as to increase the lipophilicity of Rose Bengal, and thereby modify its targeting properties in a patient.

Because the halogenated xanthenes and their derivatives are, in general, solids in their pure form, it is preferred that, for proper delivery to desired tissues, such agents be formulated in appropriate delivery vehicles. Approaches to such formulation will be generally known to those of ordinary skill in the art. Specifically, such formulations are preferred so as to facilitate agent contact with, and delivery to, desired tissues to be treated.

It is thus a further preferred embodiment of the present invention that at least one halogenated xanthene or halogenated xanthene derivative be formulated as a medicament in a topically-applicable form, such as in a liquid, semisolid, solid or aerosol delivery vehicle, including aqueous, non-aqueous or nanoparticulate suspensions, solutions, creams, ointments, gels, syrups, suppositories or micro-droplet sprays. The at least one halogenated xanthene or halogenated xanthene derivative is dissolved in such delivery vehicle, wherein this vehicle may, in addition to the at least one halogenated xanthene or halogenated xanthene derivative, include various builders, stabilizers, emulsifiers or dispersants, preservatives, buffers, electrolytes, and tissue penetrating or softening agents. Such components of the delivery vehicle may be present as the primary component (by weight or volume) of the medicament, or as a minor component that serves in an adjuvant role in agent delivery.

For example, appropriate builders include cellulose and cellulose derivatives, such as starch, and alginates.

Examples of appropriate stabilizers, emulsifiers or dispersants include liposomes, nanoparticulates and nanodispersions, microparticulates and microdispersions, as well as various lipids, detergents and other surfactants.

Examples of appropriate preservatives include benzalkonium chloride, thimerosal, and urea.

Examples of appropriate buffers include monobasic or dibasic phosphate salts, citrate salts, bicarbonate salts, and ethanolamine.

Examples of appropriate electrolytes include sodium, potassium, calcium and magnesium chlorides, phosphates, and nitrates.

Examples of appropriate tissue penetrating, softening or solvating agents and adjuvants include:
- various sulfoxides, such as DMSO and decylmethylsulfoxide;
- various aliphatic and fatty alcohols, such as ethanol, propanol, hexanoi, octanol, benzyl alcohol, decyl alcohol, lauryl alcohol, and stearyl alcohol;
- various linear and branched, saturated and unsaturated fatty acids, such as lauric acid, caproic acid, capric acid, acid, myristic acid, stearic acid, oleic acid, isovaleric acid, neopentanoic acid, trimethyl hexanoic acid, neodecanoic acid and isostearic acid;
- various aliphatic and alkyl fatty acid esters, including isopropyl n-butyrate, isopropyl n-hexanoate, isopropyl n-decanoate, isopropyl myristate, isopropyl palmitate, octyldodecyl myristate, ethyl acetate, butyl acetate, methyl acetate, methylvalerate, methylpropionate, diethyl sebacate and ethyl oleate;
- various polyols, such as propylene glycol, polyethylene glycol, ethylene glycol, diethylene glycol, triethylene glycol, diproplyene glycol, glycerol, propanediol, butanediol, pentanediol and hexanetriol;
- various amides, such as urea, dimethylacetamide, diethyltoluamide, dimethylformamide, dimethyloctamide, dimethyldecamide; biodegradable cyclic urea, such as 1-alkyl-4-imidazolin-2-one; pyrrolidone derivatives, such as 1-methyl-2-pyrrolidone, 2-pyrrolidone, 1-lauryl-2-pyrrolidone, 1-methyl-4-carboxy-2-pyrrolidone,1-hexyl-4-carboxy-2-pyrrolidone, 1-lauryl-4-carboxy-2-pyrrolidone, 1-methyl-4-methyoxycarbonyl-2-pyrrolidone, 1-methyl-4-methyoxycarbonyl-2-pyrrolidone, 1-lauryl-4-methyoxycarbonyl-2-pyrrolidone, N-cyclohexylpyrrolidone, *N*-dimethylaminopropylpyrrolidone, *N*-cocoalkypyrrolidone, N-tallowalkylpyrrolidone; biodegradable pyrrolidone derivatives, such as fatty acid esters of *N*-(2-hyroxyethyl)-2-pyrrolidone; cyclic amides, such as 1-dodecylazacycloheptane-2-one (Azone®), 1-geranylazacycloheptan-2-one, 1-farnesylazacycloheptan-2-one, 1-geranylgeranylazacycloheptan-2-one, 1-(3,7-dimethyloctyl)azacycloheptan-2-one, 1-(3,7,11-trimethydodecyl)azacycloheptan-2-one, 1-geranylazacyclohexane-2-one, 1-geranylazacyclopentan-2,5-dione, 1-farnesylazacyclopentan-2-one; hexamethylenelauramide and its derivatives; and diethanolamine and triethanolamine;
- various surfactants, such as anionic surfactants, including sodium laurate and sodium lauryl sulfate; cationic surfactants, including cetyltrimethyl ammonium bromide, tetradecyltrimethylammonium bromide, benzalkonium chloride, octadecyltrimethylammonium chloride, cetylpyridinium chloride. dodecyltrimethylammonium chloride, hexadecyltrimethylammonium chloride; nonionic surfactants, such as Polaxamer (231, 182, 184). Brij (30, 93, 96, 99), Span (20, 40, 60, 80, 85), Tween (20, 40, 60, 80), Myrj (45, 51, 52), Miglyol 840; various bile salts, such as sodium cholate, sodium salts of taurocholic, glycholic, desoxycholic acids; lecithin;
- various terpenes, including hydrocarbons, such as D-limonene, α-pinene, β-carene; various terpene alcohols, including α-Terpineol, terpinen-4-ol, carvol; various terpene ketones, including carvone, pulegone, piperitone, menthone; various terpene oxides, including cyclohexane oxide, limonene oxide, α-pinene oxide, cyclopentene oxide, 1,8-cineole; various terpene oils, including ylang ylang, anise, chenopodium, eucalyptus;
- various alkanones, such as *N*-heptane, *N*-octane, *N*-nonane, *N*-decane, *N*-undecane, N-dodecane, *N*-tridecane, *N*-tetradecane, *N*-hexadecane;
- various organic acids, such as salicylic acid and salicylites (including their methyl, ethyl, and propyl glycol derivatives), citric and succinic acid.

In addition to the examples cited here, other topically-applicable formulations familiar to those of ordinary skill in the art, including various simple or complex combinations of vehicles and adj uvants, will be useful for improving delivery of the photoactive component of the medicament to target tissues. Background on such vehicles and adjuvants may be found, for example, in: E.W. Smith and H.I. Maibach, "Percutaneous Penetration Enhancers: The Fundamentals"; S.C. Chattaraj and R.B. Walker, "Penetration Enhancer Classification"; and B.J. Aungst, "Fatty Acids as Skin Permeation Enhancers"; in E. W. Smith and H.I. Maibach (eds), Percutaneous Penetration Enhancers, CRC Press, Boca Raton, 1995.

Further, appropriate topically-applicable medicament formulations can, for example, incorporate various complex delivery vehicles, including various commercial vehicles, such as those available from Paddock Laboratories, including *Dermabase*®, *Hydrocream, Aquabase, Liquaderm-A, Liqua-Gel, Ora-Plus*®*, Ora-Sweet*® and *Ora-Sweet SF, Suspendol-S, Fattibase* and *Polybase*, as well as various proprietary vehicles, such as propylene glycol with one or more adjuvant delivery agent, so as to enhance delivery of the at least one halogenated xanthene or halogenated xanthene derivative to desired tissues to be treated. A comparison of the delivery properties for several example formulations is provided in Table 2, showing that both the quantity of active ingredient delivered to various tissues and the depth of such delivery beyond the application point can be substantially controlled by medicament formulation.

### 2. Comparative medical use of themedicaments disclosed herein for treatment of conditions affecting the skin and related organs.

The applicants have discovered that the medicaments disclosed herein are broadly applicable to improved treatment of various conditions affecting the skin and related organs of humans and animals. The medicament can be applied directly to, or substantially proximal to, tissues to be treated, including those of the skin, nails, scalp and oral cavity. Example indications include treatment for: Psoriasis and Pustular Psoriasis; Reiter's Syndrome; Skin Ulcers, including Stasis Dermatitis, Stasis Ulcers, Ischemic Ulcers, Sickle Cell Leg Ulcers, Diabetic Ulcers, Inflammatory Ulcers; Eczematous Disease and Eczematous Reaction; various Ichthyoses; Atopic Dermatitis; Superficial Wrinkles; Near Surface Fat Reduction; Benign and Malignant Proliferative Disorders, such as Benign Epithelial Tumors and Hamartomas; Premalignant and Malignant Epithelial Tumors, including Actinic Keratoses, Basal Cell Carcinoma, Squamous Cell Carcinoma, and Keratoacanthoma; Benign and Malignant Adnexal Tumors; Tumors of Pigment-Producing Cells, including Malignant Melanoma, Solar Lentigines, Nevi, and Café-au-lait; Sarcomas; Lymphomas; Vascular Disorders, such as Hemangiomas and Port Wine Stain; Microbial Infection, such as Bacterial, Fungal, Yeast, Parasitic or Other Infections; Warts; and Acne.

In a comparative example of this medical use, applicants have found that application of a cream or solution containing Rose Bengal at a concentration of approximately 0.1 % W/V to persistent leg ulcers, followed, after a latency period of 0-72 hours, and more preferably 0-1 hour, by illumination with approximately 10 to 200 J/cm² of continuous or pulsed green light in the 500-600 nm band, leads to substantial or complete healing of such persistent leg ulcers, with little or no side effects in surrounding tissue. The present invention, however, is not limited to this embodiment, as other medicaments disclosed herein can also be used. Further, other formulations of the halogenated xanthenes as described herein have similar applications for the specific indications described herein, and for various other similar indications, including those related to therapeutic or cosmetic treatment of the skin and related organs of humans and animals.

### 3. Comparative medical use of the medicaments disclosed for treatment of conditions affecting the mouth and digestive tract and related organs

The applicants have discovered that the medicaments disclosed herein are broadly applicable to improved treatment of various conditions affecting the mouth and digestive tract and related organs of humans and animals. The medicament can be applied directly to, or substantially proximal to, tissues to be treated, including those of the mouth, gums, tongue, larynx, pharynx, esophagus, stomach, intestines and colon. Example indications include treatment for: Benign Esophageal Lesions, Barretts Esophagus and other Esophageal Hyperplasia and Dysplasia, and Esophageal Cancer, including Squamous Cell Carcinoma, Adenocarcinoma, Carsinosarcoma, Pseudosarcoma, and Sarcoma; Gastric Ulcers, Leiomyomas, Polyps, Neoplasms, Lymphoma and Pseudolymphoma, Adenocarcinoma, Primary Lymphoma, Leiomyosarcoma; Oral and Oropharynx Cancer and Premalignancies, Ulcers and Inflammatory Lesions, including Squamous Cell Carcinoma, Lymphoma, Actinic Cheilitis, Nicotine Stomatitis, Leukoplakia, Erythroplakia; Gum and Other Periodontal Disease, including Gingivitis; Laryngeal Hyperplasia, Dysplasia and Neoplasms; Colorectal Cancer and Polvos.

In a comparative example of this medical use, applicants have found that application of a solution containing Rose Bengal at a concentration of approximately 1% W/V in saline to esophageal tissue, followed, after a latency period of 0-72 hours, and more preferably 0-1 hour, by illumination with approximately 10 to 200 J/cm² of continuous or pulsed green light in the 500-600 nm band, leads to substantial or complete eradication of diseased tissues, such as those present in Barretts esophagus, with little or no side effects in surrounding tissue. The present invention, however, is not limited to this embodiment, as other medicaments disclosed herein can also be used. Further, other formulations of the halogenated xanthenes as described herein have similar applications for the specific indications described herein, and for various other similar indications, including those related to therapeutic or cosmetic treatment of the mouth and digestive tract and related organs of humans and animals.

### 4. Comparative methods and medical use of the medicaments disclosed herein for treatment of conditions affecting the urinary and reproductive tracts and related organs.

The applicants have discovered that the medicaments disclosed herein are broadly applicable to improved treatment of various conditions affecting the urinary and reproductive tracts and related organs of humans and animals. The medicament can be applied directly to, or substantially proximal to, tissues to be treated, including those of the urethra, bladder, ureter, kidneys, vulva, vagina, cervix, fallopian tubes, ovaries, penis, testes, vas deferens, prostate, and epididymis. Example indications include treatment for: Urinary Tract Disease, including Cancerous and Pre-Cancerous Hyperplasia, Dysplasia and Neoplasms, Tumors and other Growths, Inflammation, and Infection of the Bladder, Ureter, Urethra, and Kidney; Cancerous and Pre-Cancerous Hyperplasia, Dysplasia and Neoplasms, Tumors and other Growths, Inflammation, and Infection of the Cervix, Endometrium, Myometrium, Ovaries, Fallopian Tubes, Uterus, Vulva, and Vagina; Cancerous and Pre-Cancerous Hyperplasia, Dysplasia and Neoplasms, Tumors and other Growths, Inflammation, and Infection of the Prostate and Testes; Reproductive Tract Infections, including Tinea Cruris, Candidiasis, Condylomata Acuminata, Molluscum Contagiosum, Genital Herpes Simplex Infection, Lymphogranuloma Venereum, Chancroid, Granuloma Inguinale, Erythrasma; Psoriasis; and Lichen Planus and Lichen Sclerosus.

In a comparative example of this medical use, applicants have found that application of a solution containing Rose Bengal at a concentration of approximately 1 % W/V to tissue, followed, after a latency period of 0-72 hours, and more preferably 0-1 hour, by illumination with approximately 10 to 200 J/cm² of continuous or pulsed green light in the 500-600 nm band, leads to substantial or complete eradication of diseased tissues, such as those present in bladder tumors, with little or no side effects in surrounding tissue. The present invention, however, is not limited to this embodiment, as other medicaments disclosed herein can also be used. Further, other formulations of the halogenated xanthenes as described herein have similar applications for the specific indications described herein, and for various other similar indications, including those related to therapeutic or cosmetic treatment of the urinary and reproductive tracts and related organs of humans and animals.

### 5. Comparative medical use of the medicaments disclosed herein for treatment of conditions affecting the respiratory tract and related organs.

The applicants have discovered that the medicaments disclosed herein are broadly applicable to improved treatment of various conditions affecting the respiratory tract and related organs of humans and animals. The medicament can be applied directly to, or substantially proximal to, tissues to be treated. Example indications include treatment for: Hyperplasia, Dysplasia and Neoplasia, Cancer, Inflammation and Infection of the Nasal Cavity, Paranasal Sinuses, Tear Ducts, Eustachian Tubes, Nasopharynx, Hypopharynx, Larynx, Trachea, Bronchi, Lung and Alveoli.

In a comparative example of this medical use, applicants have found that application of a solution containing Rose Bengal at a concentration of approximately 1% W/V to tissue, followed, after a latency period of 0-72 hours, and more preferably 0-1 hour, by illumination with approximately 10 to 200 J/cm² of continuous or pulsed green light in the 500-600 nm band, leads to substantial or complete eradication of diseased tissues, such as those present in tracheal tumors, with little or no side effects in surrounding tissue. The present invention, however, is not limited to this embodiment, as other medicaments disclosed herein can also be used. Further, other formulations of the halogenated xanthenes as described herein have similar applications for the specific indications described herein, and for various other similar indications, including those related to therapeutic or cosmetic treatment of the respiratory tracts and related organs of humans and animals.

### 6. Comparative medical use of the medicaments disclosed herein for treatment of conditions affecting various other internal or external tissue surfaces, such as tissue surfaces exposed during surgery.

The applicants have discovered that the medicaments disclosed herein are broadly applicable to improved treatment of various conditions affecting various other internal or external tissue surfaces of humans or animals, such as tissue surfaces exposed during surgery, including endoscopic surgery or other endoscopic procedures. The medicament can be applied directly to, or substantially proximal to, tissues to be treated. Example indications include treatment for: Joint Inflammation, such as that of Arthritis; Resected Tumor Beds of Intra-cranial and other Head and Neck, Thoracic, or Abdominal Tumors; Cardiac and Pericardial Tissues and Circulatory Tissues, including Arteries and Veins, including Plaques and Infections of such tissues, such as Bacterial Endocarditis; Metastatic Tumors, such as Metastases of Breast Tumors to the Skin; and various other substantially similar indications.

In a comparative example of this method of treatment or medical use, applicants have found that application of an aqueous solution containing Rose Bengal at a concentration of approximately 10 % W/V micromolar to breast adenocarcinoma and sarcoid tissues, followed, after a latency period of 0-72 hours, and more preferably 0-1 hour, by illumination with approximately 10 to 200 J/cm² of continuous or pulsed green light in the 500-600 nm band, leads to substantial or complete eradication of such tissues, with little or no side effects in surrounding tissue. The present invention, however, is not limited to this embodiment, as other medicaments disclosed herein can also be used. Further, other formulations of the halogenated xanthenes as described herein have similar applications for the specific indications described herein, and for various other similar indications, including those related to therapeutic or cosmetic treatment ofvarious other internal or external tissue surfaces of humans or animals, such as tissue surfaces exposed during surgery.

### 7. Comparative medical use of the medicaments disclosed herein for treatment of conditions related to microbial or parasitic infection.

The applicants have discovered that the medicaments disclosed herein are broadly applicable to improved treatment of various conditions related to microbial or parasitic infection of humans or animals, including those infections resistant to conventional treatments. The medicament can be applied directly to, or substantially proximal to, tissues to be treated. Example indications include treatment for: Bacterial and Antibiotic Resistant Bacterial Infection, including those caused by Gram Positives and Gram Negatives, Streptomycetes, Actinomycetes, Staphylococci, Streptococci, Pseudomonas, Escherichia coli, Mycobacteria and others; Infection caused by Filamentous Fungi and Non-filamentous Fungi like Cryptosporidium, Histoplasma, Aspergillus, Blastomyces, Candida and others; Parasitic Infection caused by Amoeba (including for use in lysing and killing amoeba in amoebic cysts), Trichinella, Dirodfilaria (Heart worm in dogs) and others.

In a comparative example of this medical use, applicants have found that application of an aqueous solution containing Rose Bengal at a concentration of approximately 1 to 10 micromolar or greater to antibiotic resistant Staphylococcus aureus, Escherichia coli, various other gram positive and gram negative bacteria, and various yeasts, followed, after a latency period of 0-72 hours, and more preferably 0-1 hour, by illumination with approximately 10 to 200 J/cm² of continuous or pulsed green light in the 500-600 nm band, leads to substantial or complete eradication of such microbes, with little or no side effects in surrounding tissue. The present invention, however, is not limited to this embodiment, as other medicaments disclosed herein can also be used. Further, other formulations of the halogenated xanthenes as described herein have similar applications for the specific indications described herein, and for various other similar indications, including those related to therapeutic or cosmetic treatment of various other conditions related to microbial or parasitic infection of humans or animals.

**Table 1. Chemical, Physical and Photochemical Properties of Some Example Halogenated Xanthenes:**

| Compound | Substitution | | | | | MW (g) | λₘₐₓ (nm) | | | α (cm⁻¹·mol ¹·L) | φ (triplet) | φ (singtel oxygen) | | |
|---|---|---|---|---|---|---|---|---|---|---|---|---|---|---|
| | x | Y | Z | R¹ | R² | | H₂O | EtOH | MeOH | | MeOH | H₂O | EtOH | MeOH |
| Fluorescein | H | H | H | Na | Na | 376 | 490 | 499 | 492 | 6.4x10⁴ | 0.03 | 0.03 | 0.03 | 0.09 |
| 4'.5'-Dichlorofluxescein | Cl | H | H | Na | Na | 445 | 502 | 511 | | | | 0.04 | 0.07 | |
| 2',7-Dichlorofluorescein | H | Cl | H | Na | Na | 445 | 502 | 511 | | | | 0.04 | 0.07 | |
| 4,5,6,7-Tetrachlorofluoresceln | H | H | Cl | H | H | 470 | 515 | | | 2.9x10⁴ | | | | |
| 2',4',5',7,T-Tetrachlorotiuoresonin | Cl | Cl | H | Na | Na | 514 | 510 | 520 | | | | 0.05 | 0.05 | |
| Dibromalluorescein | Br | H | H | Na | Na | 534 | 504 | 510 | | 1.4x10⁴ | | 0.32 | 0.42 | 0.42 |
| Solvent Red 72 | H | Br | H | H | H | 490 | | | 450 | 1.4x10⁴ | | | | |
| Diiodolluorescein | I | H | H | Na | Na | 628 | 506 | 513 | | 5.8x10⁴ | | 0.33 | 0.48 | |
| Eosin B | NO₂ | Br | H | Na | Na | 624 | 522 | | | 3.9x10⁴ | | | | |
| Eosin Y | Br | Br | H | Na | Na | 692 | 517 | 523 | 527 | 9.1x10⁴ | 0.28 | 0.32 | 0.57 | 0.39 |
| Eihyl Eosin | Br | Br | H | C₂H₅ | K | 714 | | 532 | | 1.1x10⁴ | | | | |
| Erythrosin B | I | I | H | Na | Na | 880 | 528 | 532 | 529 | 9.1x10⁴ | 0.62 | 0.69 | 0.63 | 0.62 |
| Phloxine B | Br | Br | Cl | Na | Na | 830 | 541 | 548 | 547 | 1.0x10³ | | 0.40 | 0.63 | |
| Rose Bengal | I | I | Cl | Na | Na | 1018 | 547 | 557 | 556 | 1.0x10⁵ | 0.76 | 0.86 | 0.75 | 0.76 |
| Rose Bengal Dilthium | I | I | Cl | Li | Li | 986 | | 559 | | | | | | |
| Rose Bengal Amide | I | I | Cl | C₂H₆ | (C₂H₄)₃NH | 1100 | | 563 | | | | | | 0.74 |
| Rose Bengal Diamide | I | I | Cl | (C₂H₅)₃NH | (C₂H₄)₃NH | 1166 | | 559 | | | | | | 0.72 |
| 4,5,6,7-Tetrabromoerythrosin * | I | I | Br | Na | Na | 1195 | | | | | | | | |

| | | | | | | | | | | | | | | |
|---|---|---|---|---|---|---|---|---|---|---|---|---|---|---|
| * This compound is an example falling under the scope of the claims; the other compounds are for comparison only. | | | | | | | | | | | | | | |

**Table 2. Relative delivery efficacy of comparative example transdermal delivery formulations of a halogenated xanthene; Rose Bengal is used as the agent at concentrations indicated by [RB]. The Formulation is applied to murine skin for 30 minutes under occlusive conditions. The Relative Efficacy indicates an estimate of the quantity of Rose Bengal delivered into the tissue, based on fluorescence measurement at the surface and in tissue cross sections. Depth indicates the relative depth of Rose Bengal penetration: LP = penetration to lamina propria; D+ = penetration to dermis and beyond.**

| Formulation | | | Relative Efficacy | Depth |
|---|---|---|---|---|
| \|RB\| | Vehicle | Adjuvant | | |
| 1% | Water | NaCl 09% | HIGH | LP |
| 1% | Propylene Glycol | none | LOW | LP |
| 1% | Propylene Glycol | Lauric Acid, 5% | MED-HIGH | LP |
| 1% | Propylene Glycol | Oleic Acid, 5% | MED | LP |
| 1% | Propylene Glycol | Linoleic Acid, 5% | MED-LOW | LP |
| 1% | DMSO | none | VERY HIGH | D+ |
| 1% | Liquaderm-A | none | LOW | LP |
| 1% | Liqua-Gel | none | MED-HIGH | LP |

This description has been offered for illustrative purposes only and is not intended to limit the invention of this application, which is defined in the claims below.

What is claimed as new and desired to be protected by Letters Patent is set forth in the appended claims.

## Claims

1. Use of at least one halogenated xanthene for the manufacture of a medicament for topical application, for the photodynamic treatment of diseases selected from psoriasis, pustular psoriasis, atopic dermatitis, eczematous disease and eczematous reaction;
said halogenated xanthene being dissolved in a delivery vehicle, said delivery vehicle including one or more adjuvants selected from builders, stabilizers, emulsifiers, dispersants, preservatives, buffers, electrolytes, tissue penetrating agents and tissue softening agents;
wherein said halogenated xanthene is selected from the group consisting of 4,5,6,7-Tetrabromoerythrosin and Mono-, Di-, or Tribromoerythrosin;
provided said halogenated xanthene is present in a concentration of greater than approximately 0.001% to less than approximately 20%; and
wherein said photodynamic treatment comprises activation by employing a light having a wavelength of between approximately 500 nm and 600 nm.

2. Use according to claim 1, wherein said medicament further comprises at least one targeting moiety coupled to said halogenated xanthene.

3. Use according to claim 2, wherein said targeting moiety is selected from deoxyribonucleic acids (DNA), ribonucleic acids (RNA), amino acids, proteins, ligands, haptens, carbohydrate receptors, carbohydrate complexing agents, lipid receptors, lipid complexing agents, protein receptors, protein complexing agents, chelators, encapsulating vehicles, short-chain aliphatic hydrocarbons, long-chain aliphatic hydrocarbons and aromatic hydrocarbons.

## Patentansprüche

1. Verwendung von mindestens einem halogenierten Xanthen zur Herstellung eines Medikaments zur topischen Verwendung für die photodynamische Behandlung von Erkrankungen, welche aus Psoriasis, pustulöser Psoriasis, atopischer Dermatitis, ekzematöser Erkrankung und ekzematöser Reaktion ausgewählt sind;
wobei das halogenierte Xanthen in einem Bereitstellungsvehikel gelöst wird, wobei das Bereitstellungsvehikel ein oder mehr Adjuvansien, ausgewählt aus Aufbausubstanzen, Stabilisatoren, Emulgiermitteln, Dispersantmitteln, Konservierungsstoffen, Puffern, Elektrolyten, Gewebepenetrierungsmitteln und Gewebeerweichungsmitteln, einschließt;
wobei das halogenierte Xanthen aus der Gruppe, welche aus 4,5,6,7-Tetrabromerythrosin und Mono-, Di- oder Tribromerythrosin besteht, ausgewählt ist;
mit der Maßgabe, dass das halogenierte Xanthen in einer Konzentration von höher als ungefähr 0,001 % bis niedriger als ungefähr 20 % vorhanden ist; und wobei die photodynamische Behandlung Aktivierung durch Verwenden eines Lichts mit einer Wellenlänge von zwischen ungefähr 500 nm und 600 nm umfasst.

2. Verwendung gemäß Anspruch 1, wobei das Medikament ferner mindestens eine Zielrichtungseinheit, gekuppelt an das halogenierte Xanthen, umfasst.

3. Verwendung gemäß Anspruch 2, wobei die Zielrichtungseinheit aus Deoxyribonukleinsäuren (DNA), Ribonukleinsäuren (RNA), Aminosäuren, Proteinen, Liganden, Haptenen, Kohlenhydratrezeptoren,
Kohlenhydratkomplexierungsmitteln, Lipidrezeptoren, Lipidkomplexierungsmitteln, Proteinrezeptoren, Proteinkomplexierungsmitteln, Chelatbildungsmitteln, Einkapselungsvehikeln, kurzkettigen aliphatischen Kohlenwasserstoffen, langkettigen aliphatischen Kohlenwasserstoffen und aromatischen Kohlenwasserstoffen ausgewählt ist.

## Revendications

1. Utilisation d'au moins un xanthène halogéné pour la fabrication d'un médicament pour une application topique, pour le traitement photo-dynamique de maladies sélectionnées parmi le psoriasis, le psoriasis pustuleux, la dermatite atopique, l'affection eczématique et la réaction eczématique ;
ledit xanthène halogéné étant dissous dans un véhicule de distribution, ledit véhicule de distribution comprenant un ou plusieurs adjuvants sélectionnés parmi les adjuvants, les stabilisants, les émulsifiants, les dispersants, les conservateurs, les tampons, les électrolytes, les agents de pénétration de tissu et les agents adoucissants de tissu ;
dans lequel ledit xanthène halogéné est sélectionné dans le groupe consistant en la 4,5,6,7-Tétrabromoérythrosine et la Mono-, Di- ou Tribromoérythrosine ;
pourvu que ledit xanthène halogéné soit présent en une concentration qui est supérieure à approximativement 0,001 % à moins d'approximativement 20 % ; et
dans laquelle ledit traitement photo-dynamique comprend une activation en employant une lumière dont la longueur d'onde est comprise approximativement entre 500 nm et 600 nm.

2. Utilisation selon la revendication 1, dans laquelle ledit médicament comprend en outre au moins un fragment de vectorisation qui est couplé audit xanthène halogéné.

3. Utilisation selon la revendication 2, dans laquelle ledit fragment de vectorisation est sélectionné parmi les acides désoxyribonucléiques (ADN), les acides ribonucléiques (ARN), les acides aminés, les protéines, les ligands, les haptènes, les récepteurs de glucides, les agents complexants de glucides, les récepteurs de lipides, les agents complexants de lipides, les récepteurs de protéines, les agents complexants de protéines, les chélateurs, les véhicules d'encapsulation, les hydrocarbures aliphatiques à chaîne courte, les hydrocarbures aliphatiques à chaîne longue et les hydrocarbures aromatiques.
